# EUROPEAN PATENT APPLICATION

(11) **EP 2 452 708 A1**
(43) Date of publication of application: **16.05.2012**
(21) Application number: 11182880.2
(22) Date of filing: 27.09.2011
(51) Int. Cl.: A61M 5/00, B65D 25/10

(54) **Packaging plate, syringe-holding container, and method of manufacturing combined container-syringe**

(30) Priority: 11.11.2010 JP 2010252922
(71) Applicant: ARTE CORPORATION, Tokyo (JP)
(72) Inventor: Kakiuchi, Makoto, Takahagi-shi, Ibaraki (JP); Shimazaki, Seiji, Takahagi-shi, Ibaraki (JP)
(74) Representative: De Vries & Metman

(57) **Abstract**

A packaging plate (80) that supports a plurality of syringe bodies (10) in an arranged state, including a first plate (81) in which a plurality of support hole portions into which the syringe bodies are respectively inserted and each having a rectangular shape in plan view are formed, and an extension portion (83) on which a flange portion of the syringe body is mounted is formed in each of these support hole portions; and a second plate (86) that is stacked on and fixed to the upper surface of the first plate and in which through-holes (88) through which the flange portion cannot pass are formed at locations corresponding to the support hole portions.

## Description

### BACKGROUND OF THE INVENTION

### Field of the Invention

The present invention relates to a packaging plate, a syringe-holding container, and method of manufacturing a combined container-syringe.

Priority is claimed on Japanese Patent Application No. 2010-252922, filed November 11, 2010, the content of which is incorporated herein by reference.

### Description of Related Art

Conventionally, there is known a combined container-syringe that has a function as a container that holds an injectable drug (hereinbelow called a medicinal solution), and a function of a syringe that administers a medicinal solution to a patient. Since a medicinal solution is filled in advance in the sterilized syringe body of this combined container-syringe, when used it is possible to administer the medicinal solution to a patient immediately upon removal from the packaging.

For that reason, it has many advantages of lessening the administration preparation work of busy physicians and nurses, preventing injuries and accidents, preventing the incorrect usage of medicinal drugs, preventing hospital infections, and preventing the scattering of medical agents, and so has received high praise from medical institutions. Therefore, the range of use of the combined container-syringe is rapidly expanding.

In accordance with the rising needs of the above-mentioned medical field, development is sought of a system in which, after the syringe bodies are hygienically produced in large quantities, and then packaged and distributed, the filling of the medicinal solution is efficiently performed without requiring complicated processing at a pharmaceutical company that has taken delivery of the syringe bodies.

One that has been devised for satisfying such needs of medical facilities and pharmaceutical companies is a nest-tub packaging system. In this system, after the syringe maker has manufactured the syringe bodies, they are washed and arranged in a plurality on a nest (packaging plate), and then these syringe bodies are placed in a plastic tub (syringe-holding container) in units of nests. Afterward, a sterilizable lid material is thermally bonded to the upper portion of each container, and it is delivered to the pharmaceutical company in the state of the syringe bodies inside of the container sterilized by vapor or gas.

Then, after sterilizing the periphery of the tub with hydrogen peroxide, electron rays, or the like at the pharmaceutical company, it is put in a filling apparatus, the lid material of the tub is removed, and the medicinal solution is filled once in each row of a plurality of syringe bodies that are lined up. For the syringe bodies that are filled with the medicinal solution, a stainless steel sleeve is inserted in each row of the syringe bodies at the next position in the same way, and a stopper is inserted via the sleeve, whereby the medicinal solution inside of the syringe body is sealed.

By adopting this system, there is no need for the pharmaceutical company to carry out such troublesome tasks as washing, silicon coating and sterilizing of the syringe bodies, and it can fill and seal a plurality of syringe bodies at once. Accordingly, it is possible to obtain the major advantages of a significant improvement in the production efficiency and a reduction in equipment investment.

Note that as the aforementioned nest, one disclosed for example in Patent No. 4444122 has been proposed. In this patent, a pair of claw portions of the nest fixes the flange of the syringe body, whereby it is possible to support the syringe body in a stable manner.

In the aforementioned nest-tub packaging system, there have been the following problems.

Namely, inside the tub, when a plurality of syringe bodies are in the state of being supported by the nest, as a result of handling of the tub during the distribution process, there is the risk of the syringe bodies popping out from the nest upwardly, and thus being no longer alignable.

Also, when automatically inserting metal sleeves in each row in order to set the stoppers in the syringe bodies in the filling apparatus of the pharmaceutical company, if the syringe bodies in a nest deviate even a little from the normal position, when removing the sleeves that have completed insertion of the stopper from the syringe body, they may come into contact with the side surface of the syringe body, thereby causing the syringe body to be pulled upward. Thereby, there is the risk of mechanical trouble occurring during the filling operation.

Also, the syringe body floats up not only during vertical motion of the sleeve, but also due to mechanical vibration during the filling operation of the medicinal solution, and in the case of the flange portion slipping out of the nest and not returning to its normal position, there is the risk of mechanical trouble occurring.

Moreover, in the nest disclosed in Japanese Patent Publication No. 4444122, due to the constitution that fixes the flange portion with the pair of claw portions, besides the problem of the shape of the nest itself being complicated, it is also not preferable in terms of the durability of the claw portions.

The present invention was achieved in view of the above circumstances, and has as its object to provide a packaging plate that is capable of reliably supporting syringe bodies in a state suited to the manufacture of combined container-syringes with a simple constitution, a syringe-holding container, and a method of manufacturing a combined container-syringe that uses the packaging plate.

### SUMMARY OF THE INVENTION

In order to solve the aforementioned issues, the present invention provides the following devices.

The packaging plate according to the present invention is a packaging plate that supports a plurality of syringe bodies in an arranged state, provided with a first plate in which a plurality of support hole portions into which the syringe bodies are respectively inserted and each having a rectangular shape in plan view are formed, and an extension portion on which a flange portion of the syringe body is mounted is formed in each of these support hole portions; and a second plate that is stacked on and fixed to the upper surface of the first plate and in which through-holes through which the flange portion cannot pass are formed at locations corresponding to the support hole portions.

In a packaging plate with such characteristics, the second plate is stacked on and fixed to the first plate in the state of the flange portion being mounted on the extension portion within the support hole portion of the first plate. Thereby, since the flange portion comes to exist in the space between the extension portion and the second plate in a manner incapable of slipping out, upward movement of the flange portion is blocked by the second plate. Accordingly, it is possible to prevent the syringe body from inadvertently slipping out of the support hole portion during transport or during the filling operation of the medicinal solution by the sleeve.

Also, since it is not a constitution in which the second plate itself presses down the flange portion, the flange portion can freely move in the plane direction of the first plate above the extension portion. Thereby, even if the center axis of the syringe body shifts from the normal position, it is possible to easily cause it to return to the normal position, that is, it is possible to improve the mechanical positioning accuracy when performing the medicinal solution filling work.

Moreover, since the through-holes are formed in the second plate at locations corresponding to the support hole portions of the first plate, it is possible to guide the medicinal solution or the like into the syringe body via the through-hole, and it is possible to smoothly perform manufacturing of the combined container-syringe.

Also, in the packaging plate according to the present invention, convex portions may be formed on at least one of the first plate and the second plate so as to project toward the other, and engagement hole portions with which the convex portions engage in a removable manner may be formed in the other.

In a packaging plate with such characteristics, since the first plate and the second plate are stacked and fixed in a detachable manner by the convex portions and the engagement hole portions, it is possible to easily perform lamination and fixing of the second plate to the first plate when supporting the syringe bodies, and removal of the second plate from the first plate when removing the syringe bodies.

Also, a syringe-holding container according to the present invention is provided with a container body with a box shape whose upper end is open and having a support portion that is capable of supporting the packaging plate, with the upper end opening being blocked by a lid member.

Thereby, it is possible to store and transport a plurality of the syringe bodies in a sterile state in the state of supporting these syringe bodies.

Moreover, the method of manufacturing a combined container-syringe according to the present invention is a method of manufacturing a combined container-syringe that uses the aforementioned packaging plate, including the steps of inserting the syringe body into the support hole portion of the first plate to mount the flange portion of the syringe body on the extension portion, and moreover in the state of the second plate being stacked on and fixed to the first plate, guiding a medicinal solution and a stopper into the syringe body via the through-hole of the second plate.

According to the method of manufacturing a combined container-syringe with these characteristics, since through-holes are formed in the second plate, it is possible to easily guide the medicinal solution and the stopper into the syringe body via the through hole. Also, by guiding the medicinal solution or the like into the syringe body using the packaging plate, even if the center of the syringe body shifts from the normal position, it is possible to easily cause the syringe body to return to the normal position.

According to the packaging plate and the syringe-holding container of the present invention, since they are provided with the first plate that has the support hole portions and the extension portions and the second plate that has the through-holes, it is possible to reliably support the syringe bodies in a state suited to the manufacture of combined container-syringes with a simple constitution.

Also, since the method of manufacturing a combined container-syringe of the present invention is provided with the aforementioned packaging plate, it is possible to guide a medicinal solution or the like to the syringe body via the through-hole while reliably supporting the syringe body, and it becomes possible to easily manufacture the combined container-syringe.

### BRIEF DESCRIPTION OF THE DRAWINGS

FIG. 1 is a vertical cross-sectional view of the syringe-holding container according to the embodiment of the present invention.
FIG. 2 is a vertical cross-sectional view of the combined container-syringe.
FIG. 3 is a vertical cross-sectional view of the syringe body.
FIG. 4A is a plan view of the first plate of the packaging plate according to the embodiment.
FIG 4B is a side view of the first plate of the packaging plate according to the embodiment.
FIG. 5A is a plan view of the convex portion of the first plate.
FIG 5B is a vertical cross-sectional view of the convex portion of the first plate.
FIG 6A is a plan view of the second plate of the packaging plate according to the embodiment.
FIG. 6B is a side view of the second plate of the packaging plate according to the embodiment.
FIG. 7 is a plan view that shows the state of the syringe bodies being supported by the first plate.
FIG 8A is a partial enlargement of FIG 7.
FIG 8B is a plan view that describes a modification of the extension portion.
FIG. 9 is a plan view that shows the state of supporting the syringe bodies on the first plate, and moreover the second plate being stacked on and fixed to the first plate.
FIG. 10 is a partial enlargement of FIG 9.
FIG. 11A is a drawing that describes the method of manufacture from the syringe body to the combined container-syringe.
FIG. 11B is a drawing that describes the method of manufacture from the syringe body to the combined container-syringe.
FIG. 11C is a drawing that describes the method of manufacture from the syringe body to the combined container-syringe.
FIG. 12A is a drawing that describes the method of manufacture from the syringe body to the combined container-syringe.
FIG. 12B is a drawing that describes the method of manufacture from the syringe body to the combined container-syringe.
FIG. 12C is a drawing that describes the method of manufacture from the syringe body to the combined container-syringe.
FIG. 12D is a drawing that describes the method of manufacture from the syringe body to the combined container-syringe.

### DETAILED DESCRIPTION OF THE INVENTION

Hereinbelow, an embodiment of the present invention shall be described in detail with reference to the drawings.

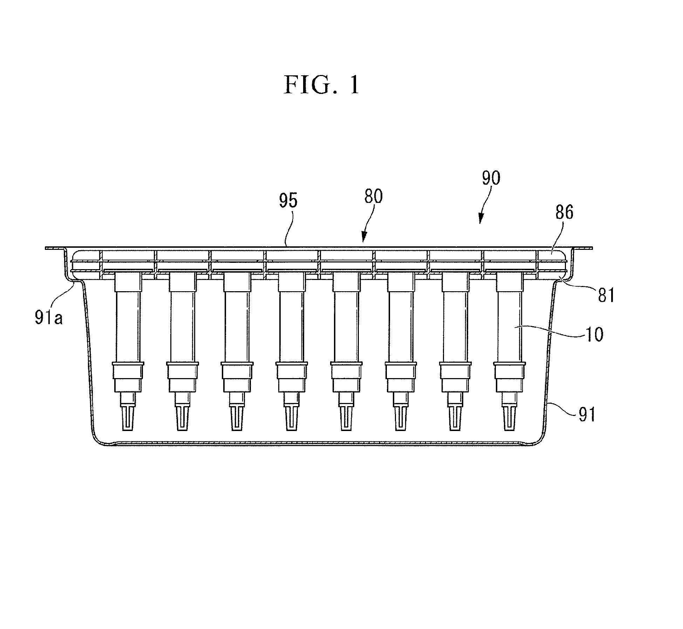

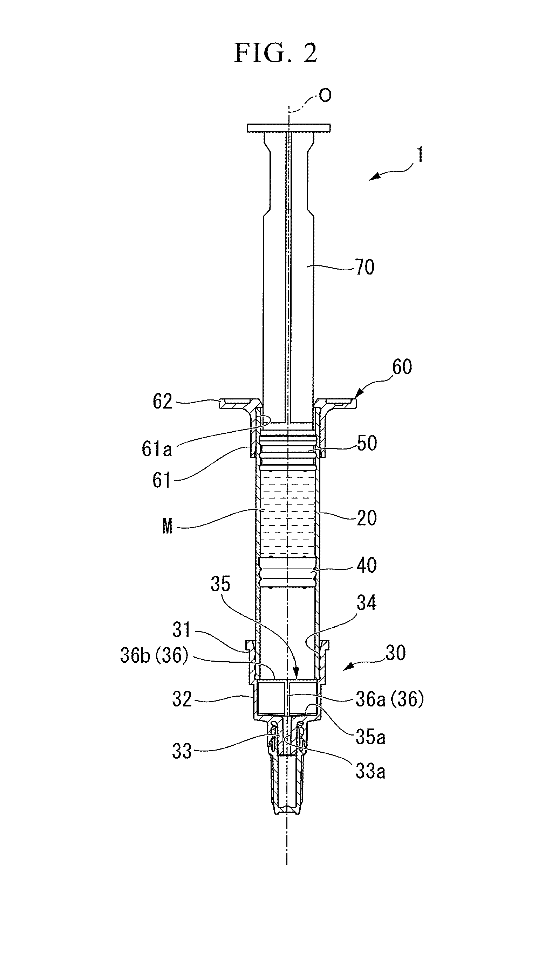

As shown in FIG. 1, a packaging plate 80 of the present embodiment is used for simultaneously supporting a plurality of syringe bodies 10 for manufacturing combined container-syringes 1 (refer to FIG. 2). In the state of the syringe bodies 10 supported by the packaging plate 80, it is possible to efficiently, safely and reliably carry out the steps of filling of a medicinal solution and sealing that is most important and difficult for the combined container-syringes 1.

First, the constitution of the combined container-syringe 1 shall be described.

The combined container-syringe 1 has a function of a container that holds a medicinal solution M and a function of administering the medicinal solution M. As shown in FIG. 2, it is provided with an outer tube 20, a tube tip 30, a front stopper 40, an end stopper 50, a finger grip 60, and a plunger rod 70.

The outer tube 20 is formed from transparent glass, and has substantially a tubular shape that extends along the center axis O.

The tube tip 30 is formed from a transparent synthetic resin that has suitable rigidity, and in contour has a multi-stage cylindrical shape centered on the center axis O. The tube tip 30 is provided with a base end portion 31 having a cylindrical shape, a cylindrical portion 32 that is coupled to the distal end side of the base end portion 31 so that the diameter is one step narrower, and a luer tip 33 that is formed at the distal end side of the cylindrical portion 32 with a smaller diameter than the cylindrical portion 32.

A fitting hole 34 that opens to the rear end side of the tube tip 30 is formed in the inner side of the base end portion 31, and a bypass chamber 35 with a bottomed hole shape is formed at the front side of the fitting hole 34, that is, the inner side of the cylindrical portion 32. The place of making contact at the bottom portion of the bypass chamber 35 is a front end surface 35a that the distal end of the front stopper 40 makes contact with. This front end surface 35a is formed with a conical surface shape that gradually decreases in diameter heading toward the front side.

Also, a guiding hole 33a that passes along the center axis O is formed inside of the luer tip 33, and one end side of this guiding hole 33a opens at the distal end of the luer tip 33, and the other end side opens at the center of the front end surface 35a in the bypass chamber 35. A syringe needle (not illustrated) that extends to the distal end side along the center axis O is attached in a continuous state at one end side of this guiding hole 33a, that is, the distal end side.

The fitting hole 34 is a hole that is formed for attaching the tube tip 30 to the outer tube 20, and the inner diameter thereof is formed to be substantially the same as the outer diameter of the outer tube 20. By causing this fitting hole 34 to fit around the distal end of the outer tube 20, the tube tip 30 can be attached to the distal end side of the outer tube 20.

The aforementioned bypass chamber 35 is a bottomed hole whose inner diameter is one step smaller than the fitting hole 34, and a bypass groove 36 is formed in the inner periphery wall. This bypass groove 36 is constituted from a linear groove 36a and an annular groove 36b.

A plurality of the linear grooves 36a are formed in the inner wall surface of the bypass chamber 35 with a regular interval in the circumferential direction so as to extend in parallel with the center axis O, and the distal end side of these linear grooves 36a extends from the inner wall surface of the bypass chamber 35 to the front end surface 35a, to be each connected to the guiding hole 33a that is formed inside of the luer tip 33.

Also, the annular groove 36b is a circular groove that extends in the circumferential direction centered on the center axis O, and is formed in the inner wall surface of the bypass chamber 35 in the vicinity of the boundary between the bypass chamber 35 and the fitting hole 34. This annular groove 36b is connected to each rear end of the plurality of linear grooves 36a, and thereby the linear grooves 36a are connected via the annular groove 36b.

The front stopper 40 and the end stopper 50 are formed from medical rubber that has corrosion resistance to medicinal solutions, and each has substantially a circular cylindrical shape centered on the center axis O having an outer diameter slightly larger than the inner diameter of the outer tube 20.

The front stopper 40 is fitted in the distal end side of the outer tube 20, and the end stopper 50 is fitted in the rear end side of the outer tube 20. Then, the medicinal solution M is sealed in the outer tube 20 by being sandwiched between the front stopper 40 and the end stopper 50.

The finger grip 60 is provided with a fitting portion 61 and a flange portion 62. The fitting portion 61 has substantially a circular cylindrical shape centered on the center axis O, and the inner periphery side thereof has a fitting hole 61 a in which the rear end of the outer tube 20 is fitted. The finger grip 60 is firmly fixed and integrated to the outer tube 20 by this fitting hole 61 a being externally fitted on the distal end of the outer tube 20.

The flange portion 62 hangs out in the diametrical direction centered on the center axis O from the rear end of the fitting portion 61, and has substantially a rectangular shape in the center axis O direction arrow view. This flange portion 62 supports the fingers of a healthcare professional during use of the combined container-syringe 1, whereby handling of the combined container-syringe 1 by the healthcare professional is made easy.

The plunger rod 70 is inserted in the outer tube 20 from the rear end of the outer tube 20 so as to pass through the finger grip 60, and the distal end of the plunger rod 70 is connected to the end stopper 50.

When using the combined container-syringe 1, the healthcare professional pushes the plunger rod 70 into the outer tube 20. Then, the end stopper 50, the medicinal solution M, and the front stopper 40 move forward, and the seal of the medicinal solution M is released at the point of the front stopper 40 having moved to the bypass chamber 35, that is to say, the medicinal solution M can be guided to the guiding hole 33a of the lure tip 33 via the bypass groove 36. Thereby, by further pushing in the plunger rod 70, it is possible to administer the medicinal solution M to a patient via a syringe needle not illustrated.

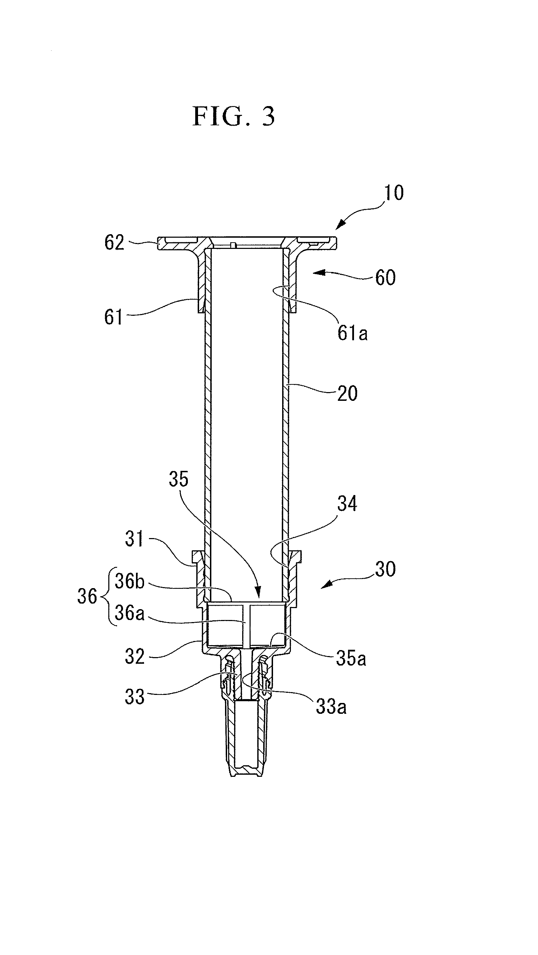

This combined container-syringe 1 is manufactured by a syringe manufacturer to the stage of the syringe body 10 shown in FIG. 3. The syringe body 10 is constituted from the outer tube 20, the tube tip 30 that is fitted on the distal end of the outer tube 20, and the finger grip 60 that is fitted on the rear end of the outer tube 20. Then, a plurality of these syringe bodies 10 are supported by the packaging plate 80 as shown in FIG. 1, and moreover transported to a pharmaceutical manufacturer in the state of being housed in a syringe-holding container 90 that includes the packaging plate 80, and the filling work of the medicinal solution M is performed at the pharmaceutical manufacturer.

Next, the constitution of the syringe-holding container 90 for housing/holding the aforementioned syringe bodies 10 in the distribution process to the pharmaceutical manufacturer shall be described.

As shown in FIG 1, the syringe-holding container 90 is provided with a container body 91, a lid member 95, and the packaging plate 80.

The container body 91 is a member that has a rectangular box shape that opens upward, and for example is formed with a synthetic resin such as plastic. The upper portion of the four side walls of the container body 91 is expanded one step toward the outside of the container body 91. Thereby, a support portion 91a that forms a flat surface facing upward is formed over the entire inner circumference of the container body 91.

The lid member 95 is a member that blocks up the upper opening of the container body 91, and is constituted from a material that causes only vapor and sterilization gas to pass. This lid member 95 is welded via a heat seal or the like to the outer circumference of the opening of the container body 91. Thereby, the sterility on the inside of the lid member 95 is maintained.

The packaging plate 80 is constituted from the two members of a first plate 81 1 and a second plate 86, as shown in FIG 1.

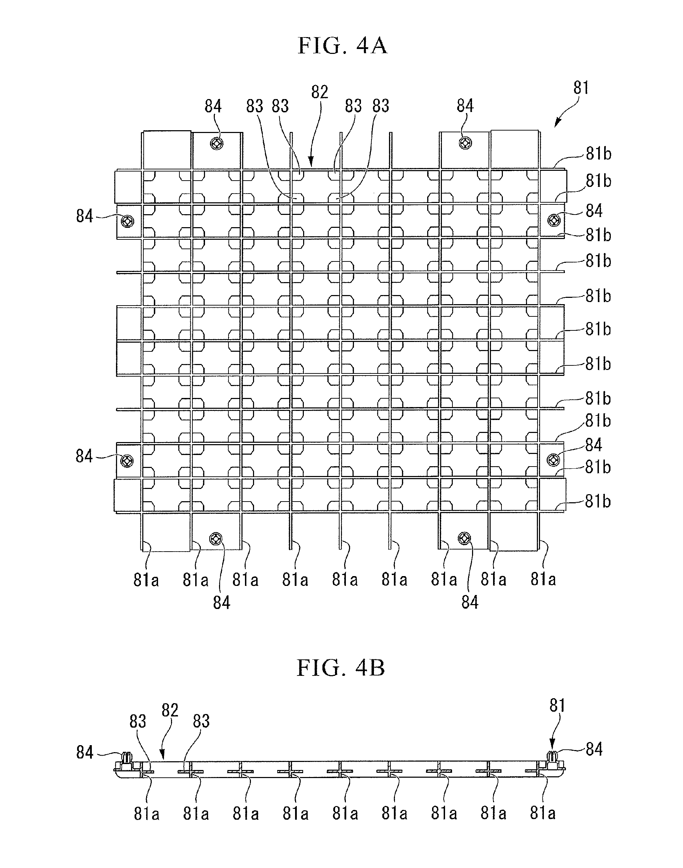

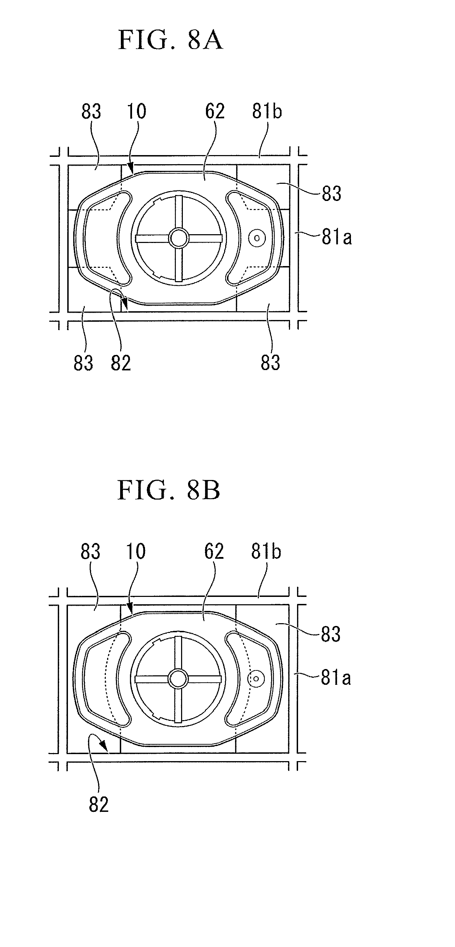

The first plate 81, as shown in FIG 4A and FIG. 8A, is a plate that forms a lattice with a rectangular shape in plan view consisting of a material such as plastic or the like, and is formed by a plurality of first plates 81a that extend in the horizontal direction and a plurality of second plates 81b that extend in a horizontal direction that is perpendicular to the first plates 81a being arranged in a lattice shape. Thereby, a plurality of rectangular spaces enclosed by the first plates 81 a and the second plates 81 b are arranged in a lattice shape.

Each rectangular space that is enclosed by the first plates 81a and the second plates 81b serves as a support hole portion 82 that is rectangular in plan view and is continuous in the vertical direction. That is, the support hole portion 82 is formed by each mutually adjacent pair of first plates 81a and each mutually adjacent pair of second plates 81 b, and the side surfaces that face the horizontal directions of these first plates 81 a and second plates 81 b serve as inner wall surfaces.

Note that the size of this support hole portion 82 in the longer-side direction in plan view is formed to be larger than the size of the flange portion 62 of the syringe body 10 in the longer-side direction when viewed from the center axis O direction. Also, the size in the shorter-side direction in plan view of the support hole portion 82 is formed larger than the size in the shorter-side direction of the flange portion 62 when viewed in the center axis O direction.

Also, an extension portion 83 that extends from the inner wall surface toward the center of the support hole portion 82 is formed at each of the four corners of the inside of the support hole portion 82 that has a rectangular shape in plan view. The four extension portions 83 in each support hole portion 82 are formed so that the fitting portion 61 of the finger grip 60 can be inserted between these four extension portions 83. That is, these four extension portions 83 have a constitution that allows insertion of the fitting portion 61 therebetween in the vertical direction while enabling mounting of the flange portion 62 on the upper surface.

Moreover, in the state of the fitting portion 61 being inserted between the four extension portions 83, a clearance is formed between the four extension portions 83 and the fitting portion 61. Thereby, in the state of the flange portion 62 being mounted on the extension portions 83, the flange portion 62 is enabled to move in the plane direction of the first plate 81 within the range of the formation region of the support hole portion 82.

Note that the shape of the extension portion 83 is not limited to the shape shown in FIG. 4A, and provided the fitting portion 61 of the finger grip 60 can be inserted in the space of the support hole portion 82, and moreover a clearance is formed in the periphery of the fitting portion 61 in this insertion state, it may also be as shown in FIG. 8B. That is, it may have a constitution in which a pair of extension portions 83 extends from the inner wall surfaces that are the shorter sides of the support hole portion 82 in plan view.

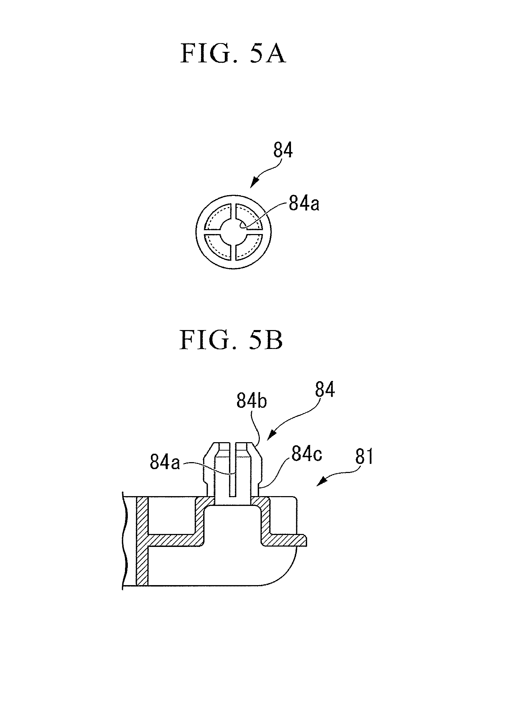

Also, a plurality of convex portions 84 that project upward may be formed on the upper surface of the outer periphery of the first plate 81. As shown in detail in FIGS. 5A and 5B, a hole portion 84a that extends from the upper part to the lower part in the center in plan view is formed in this convex portion 84, and a slit with a cross shape in plan view centered on the hole portion 84a is formed from the upper part to the lower part. At the upper portion of this convex portion 84, a tapered surface 84b whose diameter decreases toward the top is formed, and at the lower portion of this convex portion 84, a constricted portion 84c whose outer periphery surface is formed with a diameter reduced one step is formed.

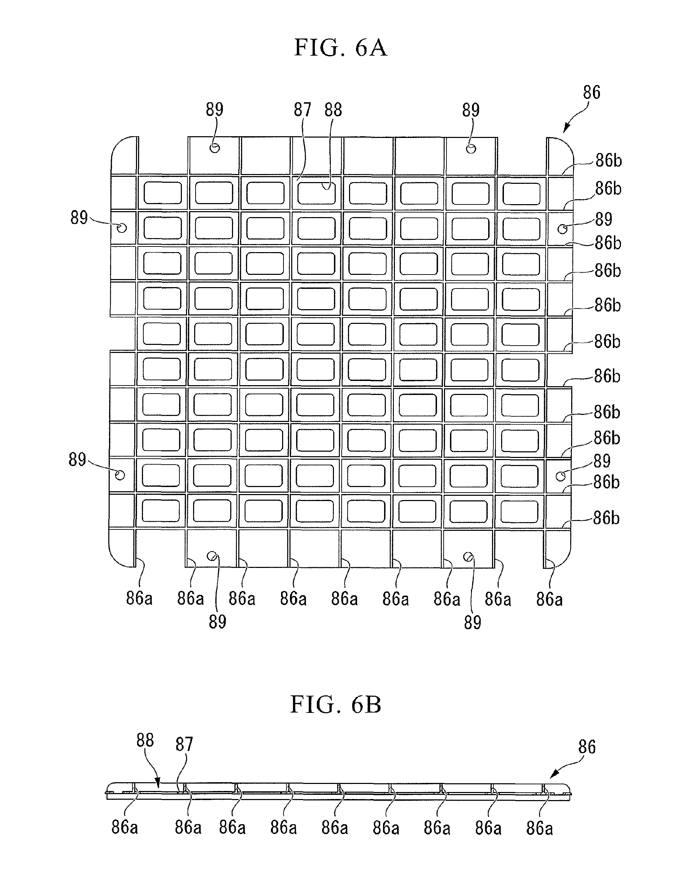

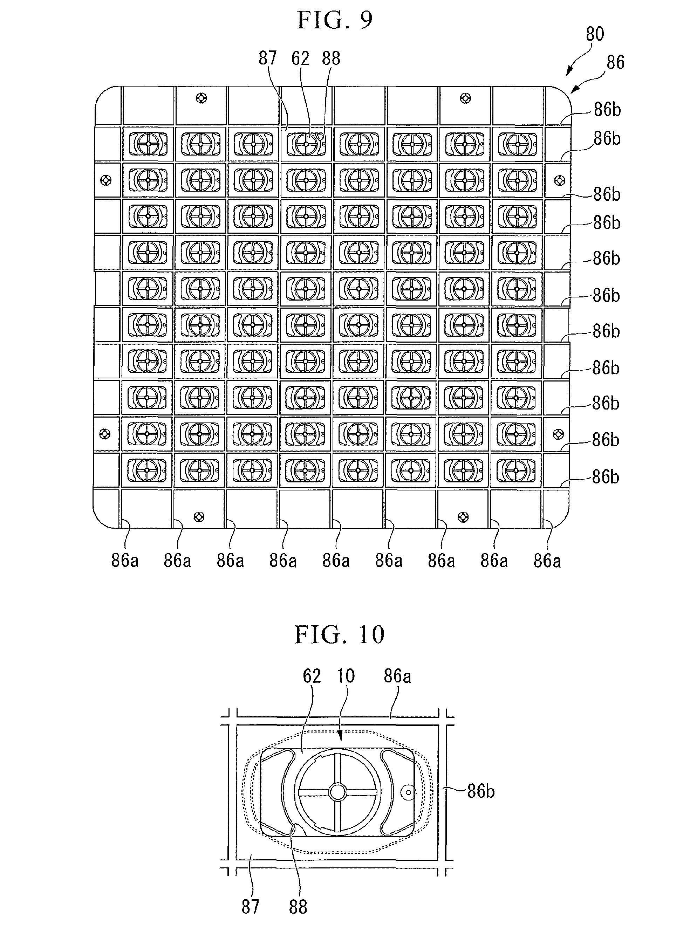

As shown in FIG. 6A and FIG. 10, the second plate 86 is a plate that forms a lattice with a rectangular shape in plan view consisting of a material such as plastic or the like, similarly to the first plate 81. Moreover, the second plate 86 is formed by a plurality of first plates 86a that extend in the horizontal direction and a plurality of second plates 86b that extend in a horizontal direction that is perpendicular to the first plates 86a being arranged in a lattice shape. Thereby, rectangular spaces enclosed by the first plates 86a and the second plates 86b are formed so as to be arranged in a plurality.

That is, each rectangular space of the second plate 86 is formed by each mutually adjacent pair of first plates 86a and each mutually adjacent pair of second plates 86b, and is in a continuous state in the vertical direction with the side surfaces that face the horizontal directions of these first plates 86a and second plates 86b serving as inner wall surfaces. Note that these rectangular spaces of the second plate 86 are arranged so as to correspond in a one-on-one relationship with the support hole portions 82 of the first plate 81.

On the inner wall surface, a perimeter plate portion 87 that hangs out from the entire circumference of the inner periphery surface is formed facing the center of the rectangular space of the aforementioned second plate 86, and a through-hole 88 with a rectangular shape is formed on the inner side of the perimeter plate portion 87. The size of this through-hole 88 in the longer-side direction in plan view is formed to be smaller than the size of the flange portion 62 of the syringe body 10 in the longer-side direction when viewed from the center axis O direction. The size of this through-hole 88 in the shorter-side direction in plan view is formed to be smaller than the size of the flange portion 62 in the shorter-side direction when viewed from the center axis O direction.

Also, engagement hole portions 89 in which the convex portion 84 can be inserted in a penetrating manner are formed on the outer periphery side of the second plate 86 at locations corresponding to the convex portions 84 of the first plate 81.

When supporting the syringe bodies 10 with this kind of a packaging plate 80, first, the first plate 81 is independently arranged in a horizontal state. Then, the distal end side of the syringe body 10, that is, the tube tip 30 side, is inserted into each support hole portion 82 from above the first plate. At this time, the tube tip 30, the outer tube 20 and the fitting portion 61 of the finger grip 60 pass through the support hole portion 82, while the flange portion 62 of the finger grip 60 makes contact with the four extension portions 83. Note that at this time, the longer-side directions and the shorter-side directions of the flange portion 62 and the support hole portion 82 are put in a state of respective agreement.

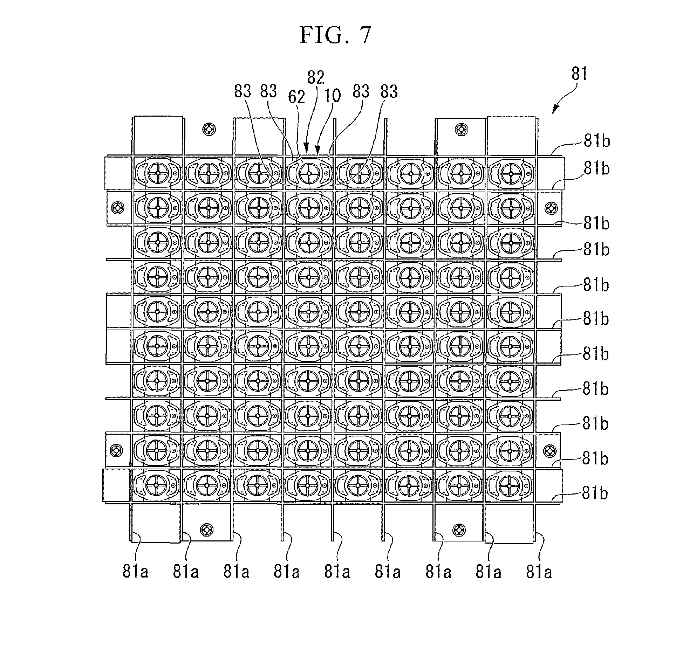

Thereby, as shown in FIG. 7 and FIG. 8A, the flange portion 62 of each syringe body 10 is put in a state of being mounted on the extension portion 83 of each support hole portion 82, and so a plurality of the syringe bodies 10 are supported in an arranged state corresponding to the formation locations of the support hole portions 82. Then, in the state of the center axis O of the syringe body 10 agreeing with the center of the support hole portion 82 (hereinbelow referred to as the normal position), each flange portion 62 forms a clearance with the inner wall surface of the support hole portion 82 in the longer-side direction and the shorter-side direction thereof. Thereby, the flange portion 62 can be moved in the range of the clearance along the upper surface of the extension portion 83, that is, along the plane direction of the first plate 81.

Then, in the state of supporting the plurality of syringe bodies 10 on the first plate 81 as described above, as shown in FIG. 9 and FIG 10, the second plate 86 is stacked on top of the first plate 81, and the convex portions 84 of the first plate 81 are inserted in the engagement hole portions 89 of the second plate 86.

At this time, due to the tapered surface 84b of the convex portion 84 being pushed from the inner periphery surface of the engagement hole portion 89, the upper portion of the convex portion 84 decreases in diameter. By this reduction in diameter, the upper portion of the convex portion 84 can pass through the engagement hole portion 89. Then, when the upper portion of the convex portion 84 has cleared the engagement hole portion 89, as a result of the constricted portion 84c of the convex portion 84 becoming engaged with the engagement hole portion 89, the convex portion 84 and the engagement hole portion 89 become mutually engaged. As a result of the convex portion 84 and the engagement hole portion 89 becoming engaged in this manner, the first plate 81 and the second plate 86 become integrally fixed.

At this time, the perimeter plate portion 87 and the through-hole 88 of the second plate 86 become arranged in a one-on-one relationship above each of the support hole portions 82 of the first plate 81.

Here, since the size of each through-hole 88 in the longer-side direction and the shorter-side direction is formed to be smaller than the size of the flange portion 62 in the longer-side direction and the shorter-side direction, the flange portion 62 cannot pass through the through-hole 88. Thereby, the syringe body 10 is supported by the packaging plate 80 so as to be incapable of slipping out.

A plurality of the syringe bodies 10 that are supported by the packaging plate 80 in this manner are housed in the container body 91 for each packaging plate 80. At this time, as shown in FIG. 1, since the outer periphery portion of the packaging plate 80 is mounted on the support portion 91a of the container body 91, the packaging plate 80 is arranged in a level state. Then, the lid member 95 is welded via a heat seal or the like to the periphery of the opening of the container body 91, whereby the syringe-holding container 90 in which the container body 91 is sealed is completed. Thereby, it is possible to store and transport a plurality of the syringe bodies 10 in a sterile state, in the state of supporting a plurality of these syringe bodies 10.

The syringe-holding container 90 that holds the syringe bodies 10 thereinside in this manner is delivered to a pharmaceutical company in this state, and is brought to a clean room or clean booth of the pharmaceutical company. Here, the lid member 95 of the container body 91 is removed, and the syringe bodies 10 are taken out from the container body 91 for each packaging plate 80, and set in a filling machine.

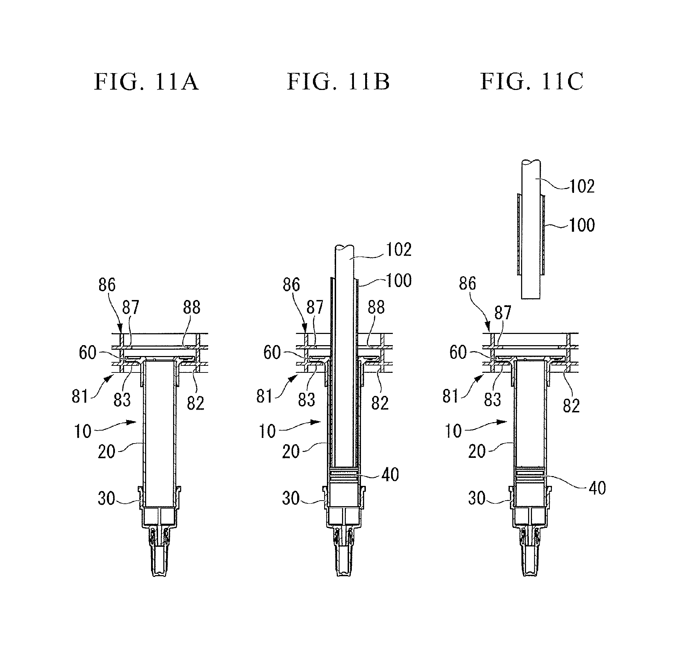

Into each of the syringe bodies 10 that are supported by the packaging plate 80 as shown in FIG. 11A, a metal sleeve 100 that has a thin-walled cylindrical shape is inserted from above as shown in FIG 11B. This metal sleeve 100 is inserted into the syringe body 10 via the through-hole 88 of the second plate 86.

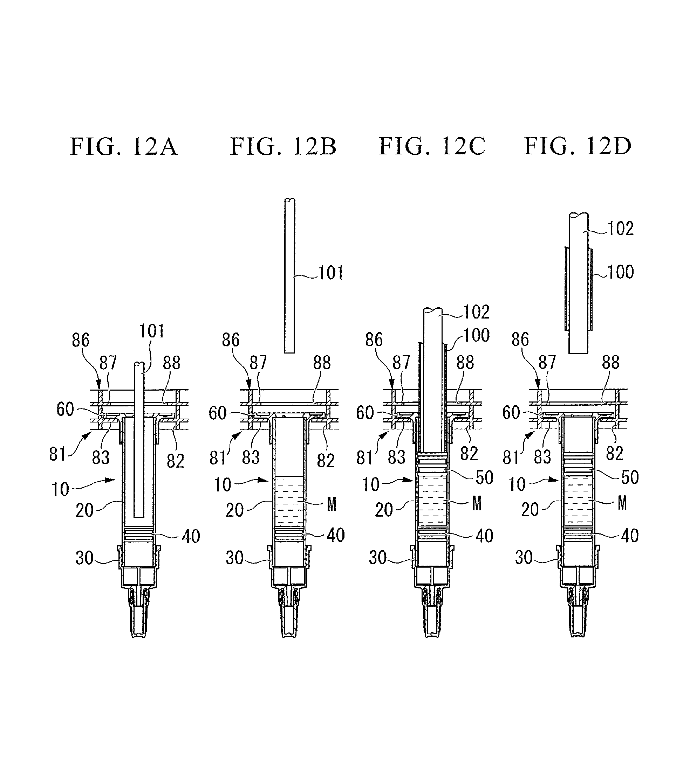

Then, as shown in FIG. 11 B and FIG 11C, the front stopper 40 is guided into the outer tube 20 through the inside of the metal sleeve 100 that has been inserted. The front stopper 40, which is pushed through the inside of the sleeve 100 while being made to contract, stops where it has been pushed by a push rod 102, and upon removal of the sleeve 100, the front stopper 40, which has been pushed out from the sleeve 100, remains in the outer tube 20. Thereafter, as shown in FIG. 12A and FIG. 12B, the medicinal solution M is guided via a nozzle 101 onto the front stopper 40 that has been inserted into the outer tube 20. This nozzle 101 is also inserted into the syringe body 10 via the through-hole 88, in the same manner as the aforementioned sleeve 100.

Next, the metal sleeve 100 is again inserted into the outer tube 20 through the through-hole 88, whereby as shown in FIG 12C and FIG. 12D, the end stopper 50 is guided into the outer tube 20 via the metal sleeve 100 and the push rod 102. In this way, the medicinal solution M is sealed by being sandwiched between the front stopper 40 and the end stopper 50 in the outer tube 20.

In this way, the sleeve 100 is inserted twice into the outer tube 20, and the front stopper 40 and the end stopper 50 are pushed in while contracting, and then pushed out into the outer tube 20 and left to remain. Accordingly, the outer diameter of the sleeve 100 must be narrower than the inner diameter of the outer tube 20 and the finger grip 60, but in order to cause the front stopper 40 and the end stopper 50 to easily pass through, it should be designed to have as large diameter as possible.

In order to mechanically insert and pull out 10 of the sleeves 100 that are designed as described above into/from 10 of the outer tubes 20 arranged in one row, the occurrence of contact with the inner surface of the finger grip 60 has been considered unavoidable. In particular, when contact occurs when withdrawing the sleeve 100 from the outer tube, the sleeve 100 is thought to lift the outer tube 20 above the packaging plate 80.

In the case of a conventional packaging plate, it has been pointed out that such a risk is high since the force that holds the outer tube 20 is weak. However, according to the present embodiment, since the finger grip 60 is firmly pressed by the second plate 86 in all of the syringe bodies 10 that have been set in the first plate 81, there is no worry of it breaking free from the first plate 81 even if there is some contact.

As described above, after the work of filling the medicinal solution M into the syringe bodies 10 is completed, the container bodies 10 are again housed within the container body 91 in the state of being supported by the packaging plate 80, and conveyed to the next stage.

In the next step, in the case of removing the syringe bodies 10 from the packaging plate 80, a plate member is inserted from both sides in the gap between the first plate 81 and the second plate 86. This plate member may be constituted so as to have a distal end that is thinner than the gap between the first plate 81 and the second plate 86, and have a thickness that increases toward the rear end side.

Thereby, the convex portions 84 of the first plate 81 disengage from the engagement hole portions 89 of the second plate 86, and the first plate 81 and the second plate 86 are separated, resulting in a state in which the syringe bodies 10 can be removed from the packaging plate 80.

Thereafter, a square bar provided with holes in which the tube tips 30 can be inserted is raised in conformity with the pitch of the arranged syringe bodies 10 from below the first plate 81. At this time, by further raising the tube tips 30 in the state of being inserted in the holes, the flange portion 62 of the syringe body 10 is made to be released from the support hole portion 82. Note that by utilizing the flange portion 62 that has slipped out at the upper portion of the first plate 81, it is possible to take out the syringe bodies 10 one by one or one row at a time from the first plate 81. Thereby, after conveying them one by one or one row at a time to the next step, and performing inspection, labeling, and the like of the filled syringe bodies 10, the plunger rod 70 is connected by being screwed into the end stopper 50, whereby the combined container-syringe 1 is completed.

As described above, the packaging plate 80 of the present embodiment is constituted by the second plate 86 being stacked on and fixed to the first plate 81 in the state of the flange portion 62 of the syringe body 10 being mounted on the extension portion 83. Thereby, since the flange portion 62 comes to be in the space between the extension portion 83 and the second plate 86 in a manner incapable of slipping out, upward movement of the flange portion 62 is blocked by the second plate 86. Accordingly, it is possible to prevent the syringe body 10 from inadvertently slipping out of the support hole portion 82 during transport or during the filling operation of the medicinal solution M by the sleeve 100.

Also, since it is not a constitution in which the second plate 86 itself presses down the flange portion 62, the flange portion 62 can freely move in the plane direction of the first plate 81 above the extension portion 83. Thereby, even if the center axis of the syringe body 10 shifts from the normal position, it is possible to easily cause it to return to the normal position, that is, it is possible to improve the mechanical positioning accuracy when performing the medicinal solution filling work.

Moreover, since the through-holes 88 are formed in the second plate 86 at locations corresponding to the support hole portions 82 of the first plate 81, it is possible to guide the medicinal solution or the like into the syringe body 10 via the through-hole 88, and it is possible to smoothly perform manufacturing of the combined container-syringe 1.

Also, the first plate 81 and the second plate 86 are fixed by the convex portions 84 and the engagement hole portions 89 which are mutually detachable. Accordingly, it is possible to easily perform lamination and fixing of the second plate 86 to the first plate 81 when supporting the syringe bodies 10, and removal of the second plate 86 from the first plate 81 when removing the syringe bodies 10.

Also, in the filling work of the medicinal solution M into the syringe body 10 using the aforementioned packaging plate 80, the through-hole 88 is formed in the second plate 86. Accordingly, it is possible to easily guide the medicinal solution M or the like into the syringe body 10 via the through-hole 88. Moreover, in the filling work of the medicinal solution M into the syringe body 10 using the aforementioned packaging plate 80, the flange portion 62 is enabled to move within the support hole portion 82 in the state of the syringe body 10 being supported by the packaging plate 80. Accordingly, even if the center of the syringe body shifts from the normal position, it is possible to easily cause the syringe body 10 to mechanically return to the normal position.

While preferred embodiments of the invention have been described and illustrated above, it should be understood that these are exemplary of the invention and are not to be considered as limiting. Additions, omissions, substitutions, and other modifications can be made without departing from the scope of the present invention. Accordingly, the invention is not to be considered as being limited by the foregoing description, and is only limited by the scope of the appended claims.

For example, in the present embodiment, the convex portions 84 were formed on the first plate 81, and the engagement hole portions 89 were formed in the second plate 86, but the convex portions 84 may be formed in the second plate 86, and the engagement hole portions 89 may be formed in the first plate 81.

## Claims

1. A packaging plate that is adapted to support a plurality of syringe bodies in an arranged state comprising:
a first plate in which a plurality of support hole portions into which the syringe bodies are adapted to be respectively inserted and each having a rectangular shape in plan view are formed, and an extension portion on which a flange portion of the syringe body is adapted to be mounted is formed in each of these support hole portions; and
a second plate that is stacked on and fixed to the upper surface of the first plate and in which through-holes through which the flange portion cannot pass are formed at locations corresponding to the support hole portions.

2. The packaging plate according to claim 1, wherein:
a convex portion is formed on at least one of the first plate and the second plate so as to project toward the other; and
an engagement hole portion with which the convex portion engages in a removable manner is formed in the other.

3. A syringe-holding container comprising:
the packaging plate according to claim 1 or 2; and:
a container body with a box shape whose upper end is open and having a support portion that is capable of supporting the packaging plate, with the upper end opening being blocked by a lid member.

4. A method of manufacturing a combined container-syringe that uses the packaging plate according to claim 1 or 2, comprising:
inserting the syringe body into the support hole portion of the first plate to mount the flange portion of the syringe body on the extension portion; and
in the state of the second plate being stacked on and fixed to the first plate, guiding a medicinal solution and a stopper into the syringe body via the through-hole of the second plate.
